# EUROPEAN PATENT APPLICATION

(11) **EP 2 772 477 A1**
(43) Date of publication of application: **03.09.2014**
(21) Application number: 12842791.1
(22) Date of filing: 01.10.2012
(51) Int. Cl.: C07C 41/26, A23L 1/226, A61K 8/34, A61Q 5/02, A61Q 11/00, A61Q 13/00, C07B 53/00, C07B 57/00, C07C 43/196, C07B 61/00

(54) **METHOD FOR PRODUCING 3-MENTHOXY PROPANOL AND COMPOSITION FOR IMPARTING COOLING SENSATION**

(30) Priority: 27.10.2011 JP 2011236075
(71) Applicant: Takasago International Corporation, Tokyo 144-8721 (JP)
(72) Inventor: TANAKA, Shigeru, Hiratsuka-shi Kanagawa 254-0073 (JP); ISHIDA, Kenya, Kanagawa 254-0073 (JP)
(74) Representative: Albrecht, Thomas
(86) International application number: PCT/JP2012/006280
(87) International publication number: WO 2013/061515

(57) **Abstract**

[Problem] The present invention provides: a composition for imparting a cooling sensation, which contains 3-menthoxy propanol; and a method for producing 3-menthoxy propanol with high selectivity and high yield using 3-menthoxy-1,2-epoxypropane as a starting material.

[Solution] The present invention relates to: a method for producing 3-menthoxy propanol with high selectivity and high yield by performing catalytic hydrogenation of 3-menthoxy-1,2-epoxypropane in the presence of a heterogeneous metal catalyst such as a cobalt, a ruthenium catalyst or a nickel catalyst; and an excellent composition for imparting a cooling sensation, which contains a mixture of 3-menthoxy-1-propanol and 1-menthoxy-2-propanol.

## Description

### Technical Field

The present invention relates to a method for producing 3-menthoxy propanol, and more specifically to a method for hydrogenating 3-menthoxy-1,2-epoxypropane in the presence of a catalyst, preferably a heterogeneous metal-supporting catalyst. 3-menthoxy propanol to be obtained by the method of the present invention is useful as an agent for imparting a cooling sensation.

### Background Art

There are various known substances which give a cooling sensation or a refreshing sensation (hereinafter these sensations may be generally referred to as "cooling and refreshing sensations") to human epidermis, mucous membranes, specifically the mouth cavity, nasal cavity, fauces, and eyes. Among them, a typical substance is 1-menthol which is a main component of peppermint oil, and 1-menthol is widely used in chewing gum, toothpastes, shampoos, and so on. 1-menthol gives strong cooling and refreshing sensations, but the effect does not last long, and the strong mint-like aroma often limits the range of applications and dose.

Therefore, for the purpose of improving the persistence and aroma, compounds giving cooling and refreshing sensations similar to those of 1-menthol are developed. Among these compounds, (1'R, 2'S, 5'R)-3-l-menthoxy alkanols, for example, 3-menthoxy-1-propanol has a marked cooling and refreshing effect, but there is still no method for producing it in a simple and low-cost manner. An example of the method for producing 3-menthoxy-1-propanol is a hydroboration method including allyl etherification of menthol, followed by addition of borane (Patent Document 1). However, the use of borane having adverse human health effects is disadvantageous for safety and cost, so that the method is not appropriate as an industrial production method.

According to another method, a heterogeneous catalyst, specifically Raney cobalt is used as the catalyst, and the epoxy ring is opened by hydrogenation to produce an alcohol (Patent Documents 2 and 3). Patent Document 2 discloses that 1,6-hexanediol is obtained at a selectivity of 60 to 74% by hydrogenating 1,2,5,6-epoxyhexane using raney cobalt as the catalyst. Patent Document 3 discloses that, when the terminal epoxide is reduced, the selectivity for the primary alcohol is about 50%, and the selectivity is about 71% and 16% or more is reduced to hydrocarbon when Raney cobalt is used as the catalyst. The selectivity is 79% at the maximum even when a Raney nickel catalyst is used, and 10% or more turns to a secondary alcohol. Therefore, the method for producing an alcohol by hydrogenation of an epoxy ring has problems that the epoxy ring can be reduced to hydrocarbon, and a large amount of secondary alcohol can be incidentally produced. However, there is no known method for producing 3-menthoxy-1-propanol with a high selectivity and a high yield by hydrogenation of 3-menthoxy-1,2-epoxypropane.

The mechanism of expression of a cooling sensation is complicated, but it is widely known that the cooling sensation is intensified by combining plural compounds giving a cooling sensation, and development of a simple method for producing the combination expressing a strong cooling sensation is desired.

### Citation List

### Patent Documents

Patent Document 1: JP 2001-294546 A
Patent Document 2: DE 10061202 A1
Patent Document 3: GB 970790 A

### Summary of Invention

### Problems to be Solved by the Invention

The present invention is intended to provide a composition for imparting a cooling sensation containing 3-menthoxy propanol which has marked functions of an agent for imparting a cooling sensation and a refreshing sensation, and a method for producing 3-menthoxy propanol with a high selectivity and a high yield using 3-menthoxy-1,2-epoxypropane as a starting material. Furthermore, the present invention is also intended to advantageously and simply adjust the ratio between the primary alcohol and secondary alcohol as the major active ingredients of the composition.

### Means for Solving the Problems

The present inventors studied to solve the problems described above, and have found that 3-menthoxy propanol is obtained with a high selectivity and a high yield by catalytically hydrogenating 3-menthoxy-1,2-epoxypropane in the presence of a heterogeneous catalyst, preferably a cobalt catalyst, a ruthenium catalyst, or a nickel catalyst, more preferably Raney cobalt. The inventors have also found that the mixture of 1-menthoxy-2-propanol and 3-menthoxy-1-propanol thus obtained works as a markedly excellent composition for imparting a cooling sensation. In addition, the inventors have also found that application range of the agent for imparting a cooling sensation is markedly extended by freely adjusting the ratio of the primary alcohol as an active ingredient, and thus have accomplished the present invention.

The present invention includes the following aspects.
[1] A method for producing 3-menthoxy-propanol including hydrogenating 3-menthoxy-1,2-epoxypropane represented by the following formula (1) in the presence of a heterogeneous metal catalyst, thereby selectively hydrogenating the epoxy ring: 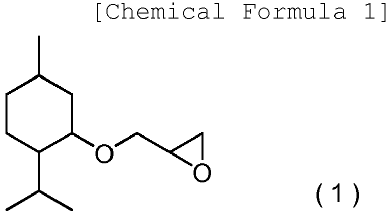
[2] The method of [1] above, wherein the heterogeneous metal catalyst is a heterogeneous cobalt catalyst, a heterogeneous ruthenium catalyst, or a heterogeneous nickel catalyst.
[3] The method of [1] or [2] above, wherein the heterogeneous metal catalyst is a Raney catalyst.
[4] The method of [3] above, wherein the heterogeneous metal catalyst is Raney nickel or Raney cobalt.
[5] The method of any one of [1] to [4] above, wherein 3-menthoxy-propanol to be obtained is a mixture of 3-menthoxy-1-propanol and 1-menthoxy-2-propanol.
[6] The method of [5] above, wherein the mixture of 3-menthoxy-1-propanol and 1-menthoxy-2-propanol contains 80% or more, preferably 90% or more of 3-menthoxy-1-propanol.
[7] The method of [6] above, wherein the heterogeneous metal catalyst is a cobalt catalyst, preferably a Raney cobalt catalyst.
[8] The method of any one of [1] to [5] above, wherein 3-menthoxy-1,2-epoxypropane represented by the formula (1) is 3-((1R,2S,5R)-2-isopropyl-5-methylcyclohexyloxy)-1,2-epoxypropane or 3-((1S,2S,5R)-2-isopropyl-5-methylcyclohexyloxy)-1,2-epoxypropane.
[9] The method of [8] above, wherein the steric configuration of the menthoxy group of 3-menthoxy-1-propanol and 1-menthoxy-2-propanol maintains the steric configuration of the starting material.
[10] The method of any one of [1] to [9] above, wherein the solvent is hexane, heptane, octane, tetrahydrofuran, or 1,4-dioxane.
[11] The method of any one of [1] to [10] above, wherein the amount of the heterogeneous metal catalyst is from 0.1% to 50% by mass in terms of the mass ratio with reference to 3-menthoxy-1,2-epoxypropane as the substrate.
[12] The method of any one of [1] to [11] above, wherein the hydrogen pressure during hydrogenation is from 1 MPa to 6 MPa.
[13] The method of any one of [1] to [12] above, wherein 3-menthoxy-propanol obtained is further subjected to separation and purification treatment to produce 3-menthoxy-1-propanol represented by the following formula (2) : 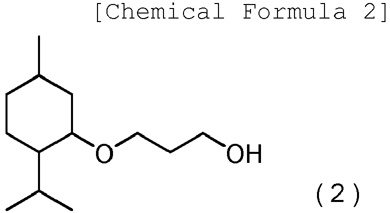
[14] The method of [13] above, wherein the separation and purification treatment is achieved by distillation.
[15] A composition for imparting a cooling sensation containing 3-menthoxy-1-propanol and 1-menthoxy-2-propanol.
[16] The composition for imparting a cooling sensation of [15] above, wherein the mass ratio between 3-menthoxy-1-propanol and 1-menthoxy-2-propanol is from 80 : 20 to 99.5 : 0.5, preferably from 90 : 10 to 99.9 : 0.1.
[17] The composition for imparting a cooling sensation of [15] or [16] above, wherein 3-menthoxy-1-propanol is 3-((1R,2S,5R)-2-isopropyl-5-methylcyclohexyloxy)-1-propanol or 3-((1S,2S,5R)-2-isopropyl-5-methylcyclohexyloxy)-1-propanol.
[18] The composition for imparting a cooling sensation of any one of [15] to [17] above, wherein 1-menthoxy-2-propanol is 1-((1R,2S,5R)-2-isopropyl-5-methylcyclohexyloxy)-2-propanol or 1-((1S,2S,5R)-2-isopropyl-5-methylcyclohexyloxy)-2-propanol.
[19] The composition for imparting a cooling sensation of any one of [15] to [18] above, wherein 3-menthoxy-1-propanol and 1-menthoxy-2-propanol is a mixture of 3-menthoxy-1-propanol and 1-menthoxy-2-propanol obtained by the method of any one of [1] to [14] above.
[20] The composition for imparting a cooling sensation of any one of [15] to [19] above, wherein the composition for imparting a cooling sensation further contains menthol, preferably 1-menthol.

### Effects of the Invention

According to the present invention, 3-menthoxy-1-propanol is industrially produced with a high selectivity and a high yield, and the steric configuration of the menthyl group maintained, and this method replaces hydroboration using borane, which is a prior art method difficult to be commercialized. It is reported that all the C-O bonds of the starting material were reduced, and 16% or more was reduced to hydrocarbon under the known hydrogenation conditions for epoxy groups (see Patent Documents 2 and 3). 3-menthoxy-1,2-epoxypropane which is a starting material in the method of the present invention has a C-O bond in the menthoxy group besides in the epoxy group, and the costly menthoxy group was expected to be reduced and fall under the prior art hydrogenation conditions for epoxy groups. On the contrary, the epoxy group was selectively reduced without reduction or falling of the menthoxy group, and a markedly advantageous industrial method for producing 3-menthoxy-propanol was provided.

In addition, according to the method of the present invention, 3-menthoxy-1-propanol is produced with a selectivity of 80% or more, preferably 90% or more, and the product contains 20% or less, preferably 10% or less of 1-menthoxy-2-propanol. However, the mixture of 3-menthoxy-1-propanol and 1-menthoxy-2-propanol produced by the method of the present invention is useful as a composition for imparting a cooling sensation giving a marked cooling and refreshing sensation without separating the components of the mixture.

Furthermore, only 3-menthoxy-1-propanol can be separated and purified from the mixture of 3-menthoxy-1-propanol and 1-menthoxy-2-propanol by distillation.

1-1-menthoxy-2-propanol is described as an exemplary compound in literatures (for example, GB 1315626 A), but there is no description about the taste and quality of the compound, or the combination with other agent for imparting a cooling sensation.

The composition for imparting a cooling sensation of the present invention containing a mixture of 3-menthoxy-1-propanol and 1-menthoxy-2-propanol has high safety, no unfavorable stimulation or bitter taste, and maintains long-lasting cooling and refreshing sensations. Therefore, the composition is widely useful as a sensational and long-lasting composition for imparting a cooling sensation for foods or beverages, aroma compositions, toiletries, and so on.

### Description of Embodiment

Preferred examples of the metal in the heterogeneous catalyst used in the method of the present invention include the metals belonging to Group 8 (Fe, Ru), Group 9 (Co, Rh, Ir), and Group 10 (Ni, Pd, Pt) of the periodic table. Specific examples of the heterogeneous catalyst include Raney nickel, ruthenium/carbon, nickel-phosphorus, nickel borate, and Raney cobalt, and particularly preferred one is Raney cobalt. The Raney cobalt catalyst is a porous and spongy metal catalyst, and may be prepared according to an ordinary method, or may be a commercially available product.

The amount of the catalyst used in the method of the present invention is from 0.1% to 50% by mass, and preferably from 1.0% to 10% by mass in terms of the mass ratio with reference to 3-menthoxy-1,2-epoxypropane as the substrate.

The present invention may be carried out in the presence or absence of a solvent, preferably in the presence of a solvent. The solvent used herein is not particularly limited, and examples thereof include hydrocarbons, alcohols such as methanol, ethanol, n-propanol, isopropanol, and n-butanol, ethers, esters, and amides such as DMF. Particularly preferred are hydrocarbon and ether solvents. Preferred examples of the hydrocarbons include aliphatic hydrocarbons such as hexane, heptane, octane, decane, cyclohexane, and methylcyclohexane, and aromatic hydrocarbons such as toluene and xylene. Examples of the ether include diisopropyl ether, methyl t-butyl ether, cyclopentyl methyl ether, cyclohexyl methyl ether, tetrahydrofuran, and 1,4-dioxane.

More preferred examples of the solvent include hexane, heptane, octane, tetrahydrofuran, and 1,4-dioxane.

The reaction time depends on the temperature, pressure, or catalyst amount, but usually from 0.5 hours to 24 hours, and preferably from 1 hour to 12 hours.

The reaction temperature depends on the pressure, catalyst amount, and solvent, but is usually from 50 to 200°C, preferably from 80 to 150°C.

The hydrogen pressure during hydrogenation is from 1 MPa to 6 MPa, and preferably from 3 MPa to 5 MPa.

After completion of the reaction, the vessel is cooled and allowed to stand, the precipitated catalyst and the reaction liquid are separated, the reaction liquid is filtered, and then the solvent is evaporated to obtain the desired 3-menthoxy-propanol. 3-menthoxy-propanol thus obtained is usually a mixture of 3-menthoxy-1-propanol and 1-menthoxy-2-propanol. 3-menthoxy-propanol produced by the method of the present invention is a mixture of 3-menthoxy-1-propanol and 1-menthoxy-2-propanol, and contains 80% or more, preferably 90% or more of 3-menthoxy-1-propanol. The mixture as it is or as necessary mixed with 3-menthoxy-1-propanol may be used as an active ingredient of the composition for imparting a cooling sensation of the present invention.

In the present invention, the coexistence of a basic substance such as a hydroxide or carbonate of an alkali metal or alkaline earth metal as a co-catalyst for the purpose of further improving the reaction selectivity may improve the selectivity and yield.

3-menthoxy-propanol obtained by the method of the present invention may be subjected to separation and purification treatment such as vacuum distillation, thereby separating and isolating 3-menthoxy-1-propanol. Alternatively, it may be a mixture with a high content of 3-menthoxy-1-propanol. When 3-menthoxy-propanol mixture is used as a component of the agent for imparting a cooling sensation of the present invention, the mixture preferably contains about 0.5 to 10% of 1-menthoxy-2-propanol.

The steric configuration of the menthoxy groups in 3-menthoxy-1-propanol and 1-menthoxy-2-propanol of the present invention is preferably the same as that of natural menthol, but may be different. According to the method of the present invention, the steric configuration of the menthoxy group of 3-menthoxy-1,2-epoxypropane used as the starting material and represented by the general formula (1) is maintained after reaction. Therefore, when the 3-menthoxy-1,2-epoxypropane having the same steric configuration as natural menthol is used as the starting material, the mixture of 3-menthoxy-1-propanol and 1-menthoxy-2-propanol having the same steric configuration as natural menthol is obtained.

The composition for imparting a cooling sensation of the present invention may be used as an agent for imparting a cooling sensation and a refreshing sensation, and also may be used as a flavor agent.

The composition for imparting a cooling sensation containing 3-menthoxy-propanol, more specifically containing the mixture of 3-menthoxy-1-propanol and 1-menthoxy-2-propanol obtained by the method of the present invention may as necessary contain other components. Examples of the other components include menthol, menthone, camphor, pulegol, isopulegol, cineole, mint oil, peppermint oil, spearmint oil, eucalyptus oil, 3-1-menthoxypropane-1,2-diol, N-alkyl-p-menthane-3-carboxamide, 3-1-menthoxy-2-methylpropane-1,2-diol, p-menthane-3,8-diol, 2-1-menthoxyethane-1-ol, 4-1-menthoxybutane-1-ol, menthyl 3-hydroxybutyrate, menthyl lactate, menthone glycerol ketal, N-methyl-2,2-isopropylmethyl-3-methylbutaneamide, menthyl glutarate, menthyl succinate, and menthyl glyoxylate. The loading of these other components is not particularly limited, and may be from 0.01 to 30 parts by mass, preferably from 0.1 to 20 parts by mass, and more preferably from 0.1 to 10 parts by mass with reference to 100 parts by mass of the composition for imparting a cooling sensation of the present invention.

In particular, the composition for imparting a cooling sensation of the present invention achieves synergistic effect with menthol, in particular with 1-menthol, so that the composition preferably contains menthol, in particular 1-menthol. The loading of menthol, in particular 1-menthol may be the above-described loading.

The composition for imparting a cooling sensation of the present invention has low volatility, almost no odor, high safety, and thus provides marked and long-lasting cooling and/or refreshing effects.

The composition for imparting a cooling sensation of the present invention may be contained in various aroma compositions, oral preparations, foods, beverages, toilet preparations (toiletries), and the like.

When the composition for imparting a cooling sensation of the present invention is added to an aroma composition such as a food or beverage aroma composition or toiletry aroma composition, the loading of the composition for imparting a cooling sensation is about 0.001 to about 95% by mass, preferably about 0.001 to about 50% by mass, and more preferably about 1 to about 40% by mass with reference to the total of the aroma composition.

When the composition for imparting a cooling sensation of the present invention is contained in an oral preparation such as, but not limited to, toothpaste, tooth powder, tooth gel, or chewing gum, the loading is about 0.001% to about 25% by mass, and preferably about 0.01% to about 15% by mass with reference to the total of the oral preparation.

When the composition for imparting a cooling sensation of the present invention is contained in a food or beverage such as, but not limited to, a fruit juice, a fruit wine, a milk drink, a carbonate drink, a sport drink, an ice cream, a sherbet, an ice candy, a jelly, or a candy, the loading is about 0.001% to about 25% by mass, and preferably about 0.01% to about 15% by mass with reference to the total of the food or beverage.

When the composition for imparting a cooling sensation of the present invention is contained in a toilet preparation such as, but not limited to, eau de toilette, a lotion, a milky lotion, a cosmetic cream, a face pack, a hair cosmetic, a shampoo, a face cleanser, an antiperspirant deodorant, or a deodorant, the loading is about 0.0001% to about 20% by mass, preferably about 0.001% to about 10% by mass, and more preferably about 0.01% to about 10% by mass with reference to the total of the toilet preparation.

The above-described aroma compositions, oral preparations, foods and beverages, and toilet preparations (toiletries) may contain any other components in addition to the composition for imparting a cooling sensation of the present invention. The choice of the additional component is known to those skilled in the art.

### Examples

The present invention is further described below in detail with reference to examples, but the present invention will not be limited to these examples.

The measurement of the product in the synthesis examples and examples was carried out using the following apparatuses.
NMR: DRX500 (manufactured by Bruker)
GC/MS: GCMS-QP2010 (manufactured by Shimadzu Co., Ltd.)
Column: RTX-1 (length 30 m x inside diameter 0.25 mm, liquid phase film thickness 0.25 µm)
GC purity: GC-14A (manufactured by Shimadzu Co., Ltd.)
Column: Chromatopac CR-4A (manufactured by Shimadzu Co., Ltd.),
Capillary Column DB-1 manufactured by J&W Scientific (length 30 m x inside diameter 0.25 mm, liquid phase film thickness 0.25 µm)
Injection temperature 250°C, detection temperature 250°C
100°C - 10°C/minute - 300°C (5 minutes)

### [Example 1]

### Production using Raney cobalt catalyst

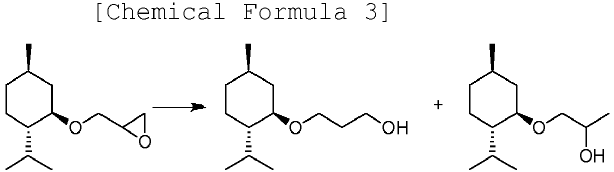

In a nitrogen gas stream, 3-l-menthoxy-1,2-epoxypropane (106 g, 0.5 mol), heptane (400 ml), and Raney cobalt (3.18 g) were placed in a 500-ml autoclave, and subjected to hydrogenation under a hydrogen pressure of 4.0 MPa, and at 130°C. After 8 hours, the decrease in the hydrogen pressure stopped, so that the analysis was carried out by GC, and the disappearance of 3-l-menthoxy-1,2-epoxypropane was confirmed. After cooling, the Raney cobalt was removed by filtration, heptane was collected under reduced pressure, and thus crude 3-l-menthoxy propanol was obtained. The crude product was subjected to vacuum distillation, and 87.7 g of a mixture of 3-1-menthoxy-1-propanol and 1-l-menthoxy-2-propanol at a mass ratio of 94 : 6 was obtained without separation.

The mixture was further subjected to vacuum distillation in a distillation column filled with 10 pieces of Sulzer Laboratory packing (diameter 30 mm x 5 cm) at 1.0 torr, and 1-l-menthoxy-2-propanol (5.00 g) with a GC purity of 98.5% was obtained from the fraction distilled out at the boiling points of 80°C to 82°C. In addition, the fraction distilled out at the boiling points of 83°C to 86°C was collected. As a result of this, 3-l-menthoxy-1-propanol (80.25 g) with a GC purity of 99.9% was obtained.

3-l-menthoxy-1-propanol
¹H-NMR (CDCl₃) : δ
0.76 (d, 3H), 0.82 (m, 1H), 0.87 (d, 3H), 0.91 (d, 3H), 0.94 (m, 1H), 1.18 (m, 1H), 1.32 (m, 1H), 1.60 (m, 2H), 1.80 (m, 2H), 2.11 (m, 2H), 2.24 (br, 1H), 3.01 (m, 1H), 3.48 (m, 1H), 3.76 (t, 2H), 3.80 (m, 1H)
¹³C-NMR (CDCl₃) : δ
16.30, 21.01, 22.36, 23.44, 25.86, 31.62, 32.53, 34.61, 40.40, 48.34, 62.48, 67.92, 79.85
GC/MS (m/e);
214 (M⁺, 3%), 199 (5), 171 (5), 155 (10), 138 (45), 129 (100), 123 (17), 109 (7), 95 (40), 81 (53), 71 (85), 55 (35), 41 (25)

1-l-menthoxy-2-propanol
¹H-NMR (CDCl₃) : δ
0.78 (d, 3H), 0.83 (m, 1H), 0.91 (d, 3H), 0.93 (d, 3H), 1.19 (d, 3H), 1.19-1.396 (m, 2H), 1.60-1.67 (m, 2H), 2.07-2.17 (br, 1H), 2.20-2.22 (m, 1H), 2.47 (d, 1H), 3.0-3.11 (m, 2H), 3.28-3.45 (m, 1H), 3.59-3.64 (m, 1H), 3.90-3.95 (m, 1H)
¹³C-NMR (CDCl₃) : δ
16.30, 18.87, 21.16, 22.51, 23.45, 25.99, 31.73, 34.75, 40.69, 48.43, 67.21, 74.28, 79.60
GC/MS (m/e);
214 (M⁺, 3%), 199 (5), 171 (5), 155 (10), 138 (65), 129 (70), 123 (20), 109 (10), 95 (30), 83 (100), 71 (50), 55 (45), 41 (30)

### [Example 2]

### Production using Raney cobalt catalyst

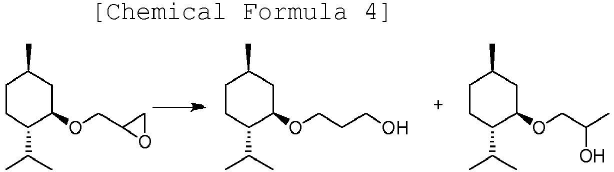

In a nitrogen gas stream, 3-1-menthoxy-1,2-epoxypropane (21.2 g, 0.1 mol), THF (100 ml), and Raney cobalt (2.0 g) were placed in a 100-ml autoclave, and subjected to hydrogenation under a hydrogen pressure of 4.0 MPa, at 130°C. After 5 hours, the decrease in the hydrogen pressure was stopped, so that the analysis was carried out by GC, and disappearance of 3-1-menthoxy-1,2-epoxypropane was confirmed. After cooling, the Raney cobalt was removed by filtration, THF was collected under reduced pressure, and thus crude 3-l-menthoxy-1-propanol was obtained. The weight ratio between 3-l-menthoxy-1-propanol and 1-menthoxy-2-propanol was 93 : 7.

The crude product was subjected to vacuum distillation, and 17.1 g of a mixture of 3-l-menthoxy-1-propanol and 1-menthoxy-2-propanol at a weight ratio of 93 : 7 was obtained without separation. The purity of the combination of 3-l-menthoxy-1-propanol and 1-menthoxy-2-propanol was 99.3% as measured by the GC analysis.

### [Examples 3 to 5]

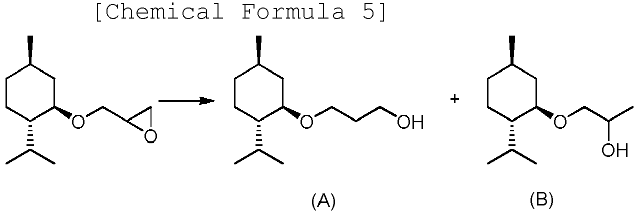

The catalyst and reaction conditions in Example 2 were changed and hydrogenation of 3-l-menthoxy-1,2-epoxypropane (2.1 g, 0.01 mol) was carried out, and thus synthesizing 3-l-menthoxy-1-propanol and 1-l-menthoxy-2-propanol.

The result is shown in Table 1. In the table, the wt% represents the weight ratio of the catalyst to 3-1-menthoxy-1,2-epoxypropane. The ml represents the amount of the solvent. The conversion rate and the product weight ratio were measured by GC. The (A) in the product weight ratio represents 3-menthoxy-1-propanol, and (B) represents 1-menthoxy-2-propanol.

**[Table 1]**

| Example | Catalyst | Wt% | Solvent | ml | Hydrogen pressure /MPa | Temperature /h | Time /h | Conversion rate/% | Product weight ratio (A) : (B) |
|---|---|---|---|---|---|---|---|---|---|
| 3 | Ni-P catakyst | 8 | THF | 20 | 6 | 150 | 20 | 100 | 80:20 |
| 4 | 10%Ru/C | 3.5 | Heptane | 4 | 3.0 | 100 | 24 | 100 | 85:15 |
| 5 | Ni₂B | 5 | Heptane | 6 | 3.0 | 120 | 20 | 100 | 80:20 |

### [Example 6]

### Comparative sensory evaluation of 3-l-menthoxy-1-propanol and the mixture of 3-l-menthoxy-1-propanol and 1-1-menthoxy-2-propanol

Using 3-l-menthoxy-1-propanol (1) obtained in Example 1, the mixture (2) of 3-l-menthoxy-1-propanol and 1-1-menthoxy-2-propanol at a weight ratio of 93 : 7 obtained in Example 2, and 1-l-menthoxy-2-propanol (3) obtained in Example 1, 50-ppm aqueous solutions were prepared.

The three 50-ppm aqueous solutions were subjected to oral sensory evaluation by ten special panelists with five or more years of experience.

As a result of this, all the ten panelists answered that (1) and (2) gave no unfavorable stimulation, and gave a marked refreshing sensation. In addition, seven of the ten panelists answered that 3-l-menthoxy-1-propanol of (1) and the mixture containing 1-l-menthoxy-2-propanol of (2) gave a stronger and long-lasting refreshing sensation. In addition, all the ten panelists answered that the intensity of cooling sensation of 1-l-menthoxy-2-propanol of (3) was 1/10 or less of that of (1), and had a stronger bitter taste.

### [Example 7]

### Synergistic effect with menthol

1-menthol and the mixture of 3-l-menthoxy-1-propanol and 1-l-menthoxy-2-propanol at a weight ratio of 93 : 7 obtained in Example 2 were mixed at a ratio of 95 : 5 (mass ratio), and thus preparing a composition for imparting a cooling sensation. Using the composition for imparting a cooling sensation thus obtained, 1,000 ml of a 20-ppm aqueous solution was prepared, and subjected to oral sensory evaluation. For comparison, a 20-ppm aqueous solution of 1-menthol alone was also subjected to oral sensory evaluation.

As a result of this, immediately after spewing out, eight of the ten panelists answered that the mixture left a stronger refreshing sensation than menthol alone.

In addition, nine of the ten panelists answered that the mixture left a stronger and long-lasting refreshing sensation than menthol alone even after a lapse of 3 minutes after spewing out.

### [Example 8]

### Toothpaste

According to the formula shown in Table 2, toothpaste was prepared. The toothpaste had no unfavorable stimulus or bitter taste, and gave a cooling sensation and had a long-lasting cooling effect.

**[Table 2]**

| (Ingredient) | (Loading g) |
|---|---|
| l-menthol | 0.25 |
| Mixture of 3-menthoxy-1-propanol and 1-menthoxy-2-propanol at a weight ratio of 93 : 7 obtained in Example 2 | 0.05 |
| Calcium hydrogen phosphate (dihydrate) | 50.00 |
| Glycerol | 25.00 |
| Sodium lauryl sulfate | 1.40 |
| Sodium carboxymethylcellulose | 1.50 |
| Saccharin sodium | 0.20 |
| Sodium benzoate | 0.10 |
| Strawberry-type flavor (manufactured by Takasago International Corporation) | 0.70 |
| Purified water | Balance |
| Total | 100.00 |

The toothpaste prepared as described above gave a cool and refreshing sensation in the mouth, and had no bitter taste. In comparison with toothpaste which did not contain 3-menthoxy-1-propanol of the present invention and a by-product 1-menthoxy-2-propanol, the toothpaste which had been prepared as described above had a long-lasting cool and refreshing flavor, and the aroma lasted a long time.

### [Example 9]

### Chewing gum

According to the formula shown in Table 3, a chewing gum was prepared. This chewing gum had no unfavorable stimulation or bitter taste, and gave a cooling sensation and had a long-lasting cooling effect.

**[Table 3]**

| (Ingredient) | (Loading g) |
|---|---|
| l-menthol | 0.01 |
| Mixture of 3-menthoxy-1-propanol and 1-menthoxy-2-propanol at a weight ratio of 93 : 7 obtained in Example 2 | 0.01 |
| Gum base | 24.00 |
| Corn syrup (42DE) | 6.70 |
| Glycerol | 1.10 |
| Sugar | 67.18 |
| Peppermint-type flavor (manufactured by Takasago International Corporation) | 1.00 |
| Total | 100.00 |

The chewing gum prepared as described above gave a cool and refreshing sensation, and had no bitter taste. In comparison with a chewing gum which did not contain 3-menthoxy-1-propanol of the present invention and the by-product 1-menthoxy-2-propanol, the chewing gum prepared as described above had a long-lasting cool and refreshing flavor, and the prolonged flavor made an exciting impression.

### [Example 10]

### Aroma composition

According to the formula shown in Table 4 (the loading is parts by mass), an aroma composition containing an agent for imparting a cooling sensation was prepared by a common procedure.

**[Table 4]**

| (Ingredient) | (Loading) |
|---|---|
| Apple base (manufactured by Takasago International Corporation) | 8.0 |
| Bergamot oil | 14.0 |
| Ethyl acetoacetate | 5.0 |
| Methyl dihydrojasmonate | 23.0 |
| Laurinal | 3.0 |
| Levosandol (manufactured by Takasago International Corporation) | 4.0 |
| Orange oil | 8.0 |
| 10-oxa-16-hexadecanolide | 8.0 |
| Phenoxanol (manufactured by IFF) | 6.0 |
| Styralyl acetate | 3.0 |
| Thesaron (manufactured by Takasago International Corporation) | 8.0 |
| Mixture of 3-menthoxy-1-propanol and 1-menthoxy-2-propanol at a weight ratio of 93 : 7 obtained in Example 2 | 30.0 |

### [Example 11]

### Shampoo

According to the formula shown in Table 5, 100 g of a shampoo containing 1.0% of the aroma composition of Example 10 was prepared. The shampoo gave a cooling sensation and had a long-lasting cooling effect.

**[Table 5]**

| (Ingredient) | (Loading g) |
|---|---|
| Sodium polyxyethylene lauryl ether sulfate | 14.00 |
| Lauric acid amidopropylbetaine | 4.00 |
| Coconut oil fatty acid diethanolamide | 3.00 |
| Cationized cellulose | 0.50 |
| Ethylene glycol distearate | 1.00 |
| Ethyl parahydroxybenzoate | 0.25 |
| Citric acid | q.l. |
| Aroma composition of Example 10 | 1.00 |
| Purified water | Balance |
| Total | 100.00 |

### Industrial Applicability

The present invention provides a novel method for producing 3-menthoxy propanol, which has marked functions as an agent for imparting a cooling sensation and a refreshing sensation, with high selectivity in a high yield. 3-Menthoxy propanol produced by the production method of the present invention is useful as an agent for imparting a cooling sensation or a refreshing sensation, and the production method and the composition for imparting a cooling sensation of the present invention have industrial applicability.

## Claims

1. A method for producing 3-menthoxy-propanol comprising hydrogenating 3-menthoxy-1,2-epoxypropane represented by the following formula (1) in the presence of a heterogeneous metal catalyst, thereby selectively hydrogenating the epoxy ring:

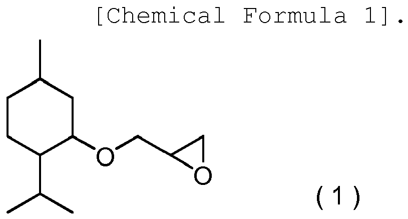

2. The method of claim 1, wherein the heterogeneous metal catalyst is a heterogeneous cobalt catalyst, a heterogeneous ruthenium catalyst, or a heterogeneous nickel catalyst.

3. The method of claim 1 or 2, wherein 3-menthoxy-propanol is a mixture of 3-menthoxy-1-propanol and 1-menthoxy-2-propanol, and the content of 3-menthoxy-1-propanol is 80% or more.

4. The method of any one of claims 1 to 3, wherein 3-menthoxy-propanol represented by the formula (1) is 3-((1R,2S,5R)-2-isopropyl-5-methylcyclohexyloxy)-1,2-epoxypropane or 3-((1S,2S,5R)-2-isopropyl-5-methylcyclohexyloxy)-1,2-epoxypropane, and the steric configuration of the menthoxy group of 3-menthoxy-1-propanol maintains the steric configuration of the menthoxy group of the 3-menthoxy-1,2-epoxypropane as a starting material.

5. The method of any one of claims 1 to 4, wherein 3-menthoxy-propanol is further subjected to separation and purification treatment to produce 3-menthoxy-1-propanol.

6. A composition for imparting a cooling sensation comprising 3-menthoxy-1-propanol and 1-menthoxy-2-propanol.

7. The composition for imparting a cooling sensation of claim 6, which further comprises menthol.
